# EUROPEAN PATENT SPECIFICATION

(11) **EP 1 047 708 B1**
(45) Date of publication and mention of the grant of the patent: **26.09.2007**
(21) Application number: 98963304.5
(22) Date of filing: 23.12.1998
(51) Int. Cl.: C07K 5/08, A61K 51/08

(54) **PEPTIDE CHELATORS THAT PREDOMINATELY FORM A SINGLE STEREOISOMERIC SPECIES UPON COORDINATION TO A METAL CENTER**
PEPTIDE CHELATOREN DIE NACH DER KOORDINATION MIT EINEM METALL ZU EINEM VORHERRSCHENDEN EINZEL-STEREOISOMEREN FÜHREN
CHELATEURS PEPTIDES CONSTITUANT POUR L'ESSENTIEL UNE SEULE ESPECE STEREOISOMERE APRES COORDINATION AVEC UN CENTRE METAL

(30) Priority: 24.12.1997 US 997802; 30.12.1997 CA 2226226
(43) Date of publication of application: 02.11.2000
(73) Proprietor: BRACCO International B.V., 1077 ZX Amsterdam (NL)
(72) Inventor: POLLAK, Alfred, Toronto, Ontario M6B 4C6 (CA); FAUCONNIER, Theresa, Toronto, Ontario M4K 2X6 (CA); WONG, Ernest, Langley, British Columbia V2Y 1N5 (CA)
(74) Representative: Bassil, Nicholas Charles
(86) International application number: PCT/CA1998/001201
(87) International publication number: WO 1999/033863

(56) References cited:
- EP-A- 0 284 071
- WO-A-95/22996
- WO-A-95/33497
- WO-A-96/03427
- WO-A-96/40293
- S.H. PEERS ET AL.: "Imaging a model of colitis with RP128, a Tc-99m-chelated tuftsin antagonist" THE JOURNAL OF NUCLEAR MEDICINE, PROCEEDINGS OF 42ND ANNUAL MEETING, vol. 36, 15 June 1995, page 114 XP002102963
- WONG, ERNEST ET AL: "Rhenium(V) and Technetium(V) Oxo Complexes of an N2N'S Peptidic Chelator: Evidence of Interconversion between the Syn and Anti Conformations" INORG. CHEM. (1997), 36(25), 5799-5808 CODEN: INOCAJ;ISSN: 0020-1669,1997, XP002102964

## Description

### Technical Field

This invention relates to chelators that form a mixture enriched for a single stereoisomeric species upon coordination to a metal center.

### Background of the Invention

The current interest in radiolabeling biologically important molecules (proteins, antibodies, and peptides) with ^{99m}Tc stems from the desire to develop a target specific diagnostic radiopharmaceutical.^{1-10.} The advantages of using ^{99m}Tc in diagnostic nuclear medicine are well known¹¹⁻¹⁵ and a number of techniques have been developed for the ^{99m}Tc labeling of biologically important molecules.¹⁶⁻²⁰

One obvious approach is to coordinate a ^{99m}Tc metal directly with the targeting molecule. This approach is known as the direct labeling method and it involves the use of a reducing agent to convert disulfide linkages into free thiolates, which then bind to the ^{99m}Tc metal. A major disadvantage of this method is the lack of control over the coordination of the ^{99m}Tc metal and the stability of the resulting metal complex. In addition, the lack of suitable or accessible coordination sites in some proteins and peptides exclude direct labeling as a viable technique.

Two common alternatives to direct labeling are the final step labeling method and the pre-formed chelate method. Both techniques involve the use of a bifunctional chelator, which provides the site of ^{99m}Tc coordination. The difference between the two methods lies in the order in which the ^{99m}Tc complex is formed. In the final step labeling method, complexation occurs after the chelator has been attached onto the targeting molecule. With the pre-formed chelate method, the ^{99m}Tc complex is initially prepared and purified before being attached to the targeting molecule. In both methods, the bifunctional chelator must coordinate to ^{99m}Tc to form a complex that is stable *in vivo* and the chelator must have an active moiety that can react with a functional group on the targeting molecule.

A number of bifunctional chelators have been used in the labeling of proteins, peptides and monoclonal antibodies.^{2, 9, 10, 17, 21-28} Depending on the chelator, the labeling of biologically important molecules with bifunctional chelators often results in the formation of multiple species or isomeric complexes. An example is the ^{99m}Tc labeling of molecules using the hydrazinonicotinamide (HYNIC) system. Since the HYNIC group can only occupy one or two sites of Tc coordination, co-ligands are required to complete the coordination sites. Glucoheptonate²⁹⁻³⁰, tris(hydroxymethyl)methylglycine (tricine)²⁵, ethylenediamine-N, N'-diacetic acid (EDDA)⁹, water soluble phosphines²⁵ [trisodium triphenylphosphine-3,3',3"-trisulfonate (TPPTS), disodium triphenylphosphine-3,3'disulfonate (TPPDS), and sodium triphenylphosphine-3-monosulfonate (TPPMS)] and polyamino polycarboxylates⁹ have all been used as co-ligand in the HYNIC system.

It has been clearly shown that the Tc-99m labeling of molecules via the HYNIC/co-ligand system produces multiple species, which is due to the different coordination modalities of the hydrazine moiety and the co-ligands. The number of species, the type, the stability and the properties of the species vary greatly from one co-ligand to another. In the labeling of chemotactic peptides using the HYNIC system, the nature of the co-ligand also greatly affects the biodistribution of the labeled peptide.³¹

Another example of a bifunctional chelator producing multiple species is dithiosemicarbazone (DTS) system. It has been shown that the DTS bifunctional chelator produces at least four complexes with technetium.³² Two of the complexes are known to be charged; hence they have different biodistribution from the uncharged species.

As in the development of a pharmaceutical based on organic molecules, the stereochemistry or isomerism of a metal complex is very important in the development of a radiopharmaceutical or metallodrug. It is well known that isomers can have different lipophilicities, biodistribution patterns, and biological activities. An example of this is the ^{99m}Tc complex of 3,6,6,9-tetramethyl-4,8-diazaundecane-2,10-dione dioxime (^{99m}Tc-d,l-HMPAO or Ceretec), which is a cerebral perfusion imaging agent.^{14,33-35} Though ^{99m-}Tc-d,1-HMPAO is active, it has been shown that the meso analogs of the ^{99m}Tc HM-PAO^{14,36} complex and the ^{99m}Tc complex of 3,3,9,9-tetramethyl-4,8-diazaundecane-2,10-dione dioxime^{14,37} (PnAO) do not possess the properties necessary for use as a cerebral perfusion imaging agent

A type of Tc and Re coordination modality common in Tc and Re radiopharmaceuticals is the coordination of a tetradentate N₄₋ₓSₓ chelator to a metal oxo moiety to form a square pyramidal or octahedral metal oxo complex. A host of bifunctional chelators have been developed based on the tetradentate N₄₋ₓSₓ coordination motif. Examples include N₄ propylene amine oxime³⁸, N₃S triamide thiols^{9, 39-43}, N₂S₂ diamide dithiols^{9, 44-46}, N₂S₂ monoamide monoaminedithiols⁴⁷⁻⁴⁹ and N₂S₂ diamine dithiols⁵⁰⁻⁵⁵. Functionalization of the chelator backbone enables these chelators to be attached to biologically interesting molecules. The labeling of these bifunctional chelators with TcO³⁺ or ReO³⁺ often produces isomers or epimers.^{39-43, 46-55} The isomers or epimers (*syn* and *anti*) arise from the configuration of the metal oxo group relative to the functional group on the chelator backbone. It has been clearly shown that the biodistribution and biological activity of the *syn* and *anti* isomers are often different.^{39-43, 46, 56} The Tc complex of mercaptoacetylglycylglycylglycine (MAG₃), a renal imaging agent, exists in the *syn* and *anti* isomers. The biological activities of the *syn* and *anti* isomers are known to be different.^{39,40} The *syn* and *anti* isomers of the Tc complex of 2,3-bis(mercaptoacetamide)propanoate (map) wwere also shown to have different biological activities.⁴⁶ It was reported that, in humans, 58% of the *syn* isomer was excreted at 30 minutes as compared to only 19 % of the *anti* isomer. Another example of the isomers exhibiting a difference in biological bebaviour is the ^{99m}Tc labeled diamino dithiol piperidine conjugates, which were investigated as brain perfusion imaging agents. It was shown that the two isomeric complexes exhibit widely disparate brain uptake.⁵⁵ At 2 minute post-administration in rats, uptake of the *anti* isomer in the brain was 1.08 % dose/g, while the uptake of the *syn* isomer was 2.34 % dose/g. The brain/blood ratio at 2 minute post-administration was 2.09 for the *anti* isomer and 5.91 for the *syn* isomer.
The peptide dimethylglycine-L-serine-L-cysteine-glycine is a bifunctional chelator that can be used to label biologically important molecules.^{61,62} It has been shown that dimethylglycine-L-serine-L-cysteine-glycine coordinates to TcO³⁺ and ReO³⁺ via a monoamine diamide monothiol coordination modality.⁶¹ The resulting Tc and Re complexes exist as two isomers; the serine CH₂OH side chain is in the *syn* and *anti* conformations with respect to the metal oxo bond. The presence of the *syn* and *anti* isomers is very evident from the NMR spectral data. In the ¹H NMR spectrum of the Re complex, there were two pairs of singlets associated with the nonequivalent methyl groups in the dimethylglycine residue. Each pair of singlets corresponded to either the *syn* or *anti* isomers. The presence of the two isomers is clearly evident from the NMR data. In the coordination of dimethylglycine-L-isoleucine-L-cysteine-glycine (RP349) to ReO³⁺, two isomers (*syn* and *anti*) were also observed. The ^{99m}Tc labeling of RP294 and RP349 produced *syn* and anti isomers; two peaks were observed in the HPLC using the radiometric detector. The ^{99m}Tc labeling of biotin with dimethylglycine-L-lysine-L-cysteine-NH₂ (RP332) also produced *syn* and anti isomers; two peaks were observed in the HPLC. These results are consistent with the coordination of other tetradentate N₄₋ₓSₓ chelators to TcO³⁺ and ReO³⁺.^{9,39-55}

The labeling of biologically important molecules via a bifunctional chelator can result in the formation of isomers or multiple species, which can have significant impact on the biological properties of the radiopharmaceutical. For receptor-based radiopharmaceuticals, the target uptake is largely dependent on the receptor binding affinity of the targeting molecule and the blood clearance of the labeled molecule, which is determined by the physical properties of both the targeting molecule and the metal chelate. Hence, the presence of isomers for the metal chelate can have significant impact on the radiopharmaceutical. Therefore, in the development of a radiopharmaceutical or metallodrug, it is necessary to separate the isomers and evaluate the biological activities of each individual isomer. It would therefore be desirable to develop chelators that predominately form a single stereoisomeric species upon coordination to a metal center.

An article entitled Imaging a Model of Colitis with RP128, A Tc-99m Chelated Tuftsin Antagonist by S.H. Peers et al (The Journal of Nuclear Medicine, Proceedings of 42nd Annual Meeting, vol. 36., 15 June 1995, page 114, XP002102963) discloses the ability of Tc99M-RP128 to image rat model inflammatory disease. The article does not discuss isomerization of the targeting agent.

An article entitled Rhenium (V) and Technetium (V) Oxo Complexes of an N₂N'S Peptidic Chelator: Evidence of Interconversion Between the Syn and Anti Conformations by Wong et al (Inorg Chem. (1997), 36(25), 5799-5808) shows the radiolabelling of oxo complexes of the peptide RP294. Although interconversion between syn and anti isomers is shown, a compound that predominately forms a single isomer upon coordination to a metal center is not disclosed.

European Patent Application 0284071 discloses chelated radionuclide compositions that are provided for conjugation to polypeptides and carbohydrates.

International Publication Number WO 95/33497 describes radiopharmaceuticals for targeting sites within a mammalian body. In particular, the radiopharmaceuticals comprise targeting molecules that are covalently linked to monoamine, diamine, thio-containing metal chelators.

International Publication Number WO 96/40293 describes metallo-constructs that can be used for diagnostic imaging and therapy.

International Publication Number WO 95/22996 describes peptide-chelator conjugates that are useful for diagnostic imaging of sites of inflammation.

International Publication Number WO 96/03427 describes peptide derived radionuclide chelators for use in imaging sites of diagnostic interest within the body.

### Summary of the Invention

Chelators and chelator-targeting molecule conjugates are provide that form a mixture with a predominant stereoisomeric species upon coordination to a metal center.

According to an aspect of the invention, there is provided a compound that predominately forms a single stereoisomer upon coordination to a metal center of the following formula I: 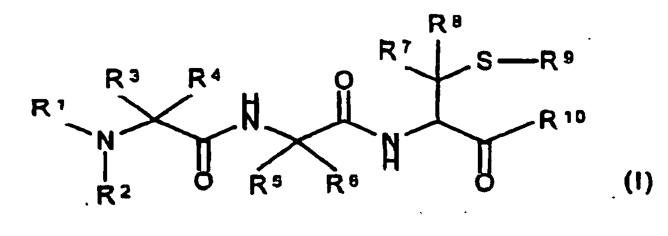 wherein
R¹ is a linear or branched, saturated or unsaturated C₁₋₄alkyl chain that is optionally interrupted by one or two heteroatoms selected from N, O and S; and is optionally substituted by one or more substituents selected from halogen, hydroxyl, amino, carboxyl, C₁₋₄alkyl, aryl and C(O)R¹⁰;
R² is H or a substituent defined by R¹;
R¹ and R² may together form a 5- to 8-membered saturated or unsaturated heterocyclic ring optionally substituted by one or more substituents selected from halogen, hydroxyl, amino, carboxyl, oxo, C₁₋₄alkyl, aryl;
R³, R⁴ and R⁵ are selected independently from H; carboxyl; C₁₋₄alkyl; C₁₋₄alkyl substituted with a substituent selected from hydroxyl, amino, sulfhydryl, halogen, carboxyl, C₁₋₄alkoxycarbonyl and aminocarbonyl; an alpha carbon side chain of a D- or L-amino acid other than proline; and C(O)R¹⁰;
R⁶ is selected from a group consisting of ;
   i) an optionally subsituted 3- to 6-membered heterocylic or carbocylic ring,
   ii) a compound of the following formula: 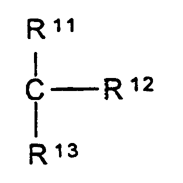 wherein R¹¹, R¹² and R¹³ are independently selected from H, linear or branched, saturated or unsaturated C₁₋₆alkyl chain that is optionally interrupted by one or two heteroatoms selected from N, 0 and S; and is optionally substituted by one or more substituents; alkoxycarbonyl, aminocarbonyl, alkoxy, an optionally subsituted 3- to 6-membered heterocylic or carbocylic ring; with the proviso that a least one of R¹¹, R¹² and R¹³ is not H;
   iii) a compound of the following formula: 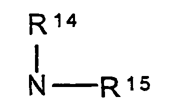 wherein R¹⁴ and R¹⁵ are independently selected from H, linear or branched, saturated or unsaturated C₁₋₆alkyl chain that is optionally interrupted by one or two heteroatoms selected from N, O and S; and is optionally substituted by one or more substituents (; alkoxycarbonyl, aminocarbonyl, alkoxy, an optionally subsituted 3- to 6-membered heterocylic or carbocylic ring; with the proviso that a least one of R¹⁴ and R¹⁵ is not H;
   and iv) a compound of the following formula: 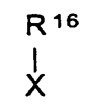 wherein X is selected from O or S and R¹⁶ is selected from linear or branched, saturated or unsaturated C₁₋₆alkyl chain that is optionally interrupted by one or two heteroatoms selected from N, O and S; and is optionally substituted by one or more substituents; alkoxycarbonyl, aminocarbonyl, alkoxy, and an optionally subsituted 3- to 6-membered heterocylic or carbocylic ring,
R⁷ and R⁸ are selected independently from H; carboxyl; amino; C₁₋₄alkyl; C₁₋₄alkyl substituted by a substituent selected from hydroxyl, carboxyl and amino; and C(O)R¹⁰;
R⁹ is selected from H and a sulfur protecting group; and
R¹⁰ is selected from hydroxyl, alkoxy, an amino acid residue, a linking group and a targeting molecule; and
wherein the metal center is selected from technetium or rhenium.

According to another aspect of the invention, there is provided a compound that predominately forms a single stereoisomer upon coordination to a metal center of the following formula II: 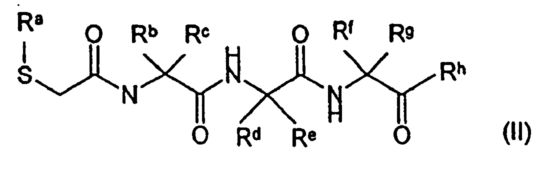 wherein
R^{a} is selected from H and a sulfur protecting group;
R^{b}, R^{c} R^{d}, R^{f} and R^{g} are selected independently from H; carboxyl; C₁₋₄alkyl; C₁₋₄alkyl substituted with a substituent selected from hydroxyl, amino, sulfhydryl, halogen, carboxyl, C₁₋₄alkoxycarbonyl and aminocarbonyl; an alpha carbon side chain of a D- or L-amino acid other than proline; and C(O)R^{h};
R^{e} is selected from a group consisting of:
   an optionally subsituted 3- to 6-membered heterocylic or carbocylic ring; and 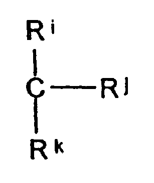
   wherein Rⁱ, R^{j} and R^{k} are independently selected from H, linear or branched, saturated or unsaturated C₁₋₆alkyl chain that is optionally interrupted by one or two heteroatoms selected from N, O and S; and is optionally substituted by one or more substituents; alkoxycarbonyl, aminocarbonyl, alkoxy, an optionally subsituted 3- to 6-membered heterocylic or carbocylic ting; with the proviso that a least one of Rⁱ, R^{j} and R^{k} is not H; and 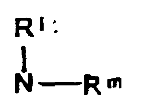 wherein R^{l} and R^{m} are independently selected from H, linear or branched, saturated or unsaturated C₁₋₆alkyl chain that is optionally interrupted by one or two heteroatoms selected from N, O and S; and is optionally substituted by one or more substituents; alkoxycarbonyl, aminocarbonyl, alkoxy, an optionally substituted 3- to 6-membered heterocylic or carbocylic ring; with the proviso that a least one of R^{l} and R^{m} is not H; and 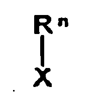 wherein X is selected from O or S and Rⁿ is selected from linear or branched, saturated or unsaturated C₁₋₆alkyl chain that is optionally interrupted by one or two heteroatoms selected from N, O and S; and is optionally substituted by one or more substituents; alkoxycarbonyl, aminocarbonyl, alkoxy, and an optionally subsituted 3- to 6-membered heterocylic or carbocylic ring; and
R^{h} is selected from hydroxyl, alkoxy, an amino acid residue, a linking group and a targeting molecule; and
wherein the metal center is selected from technetium or rhenium.

According to another aspect of the invention, the chelator-targeting molecule conjugates are provided in combination with a diagnostically useful metal or an oxide or nitride thereof, wherein the metal center is selected from technetium or rhenium.

According to a further aspect of the invention there is provided a pharmaceutical composition comprising a pharmaceutically acceptable carrier and a compound as described above in an amount effective to image a site of diagnostic interest.

According to another aspect of the present invention, there is provided a pharmaceutical composition as described above for use in imaging a site of diagnostic interest.

### Detailed Description of the Invention

In the coordination of dimethylglycine-*t*-butylglycine-cysteine-glycine to TicO³⁺ and ReO³⁺, a single isomer was observed. A single pair of singlets associated with the methyl groups in the dimethylglycine residue was observed. The ^{99m}Tc labeling of dimethylglycine-L-*t*-butylglycine-L-cysteine-glycine (RP455) and of dimethylglycine-D-*t*-butylglycine-L-cysteine-glycine (RP505) produced a single peak as observed in the HPLC using the radiometric detector. This was an unexpected result and contrasted with what was observed in the Tc and Re oxo complexes of other tetradentate N₄₋ₓSₓ chelators,^{9, 39-55} which existed as the *syn* and *anti* isomers.

The presence of a sterically bulky group in the side chain of the peptidic chelator caused the formation of a single isomeric metal complex. In the cases of dimethylglycine-L-lysine-L-cysteine and dimethylglycine-L-serine-L-cysteine-glycine, there was insufficient bulk to cause one isomer to be preferred over another; hence the ratio of the *syn* and *anti* isomers was approximately 1:1.

In the case of dimethylglycine-L-isoleucine-L-cysteine, a more sterically bulky CH(CH₃)-CH₂-CH₃ group was introduced into the peptidic backbone. This additional bulk caused the ratio of the *syn* and *anti* isomers to be 3:1; hence, one isomer was more favored over the other. In the case of dimethylglycine-*t*-butylglycine-cysteine-glycine, the incorporation of the C(CH₃)₃ group introduced sufficient bulk into the peptide to cause one of the isomers to be completely favored over the other; hence, a single isomeric metal complex was observed.

Molecular modeling with Quanta Charm indicated that the *syn* isomer was favoured because in the *anti* isomer there was steric interaction between the bulky side group and the oxygen atoms of the adjacent amide groups. For example, the dihedral angles of the beta carbon of serine with the backbone of the chelate in the *anti* isomer of the Re complex of dimethylglycine-L-serine-L-cysteine-glycine (ReORP414) were - 27-39° (O-C-C-C) and 8.35° (C-C-N-C). The corresponding dihedral angles for the *anti* isomer of the Re complex of dimethylglycine-L-t-butylglycine-L-cysteine-glycine (ReORP455) were -11.95° and -6.87°. The difference of about 15° for each angle was a result of the shift of the amide oxygen atoms and the side group atoms of ReORP455 to a position of least contact. The shift of atomic positions induced some strain on the chelate system and therefore lessened its stability.

Molecular modeling of each of the Re complexes of the peptides was in agreement with experimental results. Molecular modeling of the Re complex of dimethyglycine-L-serine-L-cysteine-glycine showed the two isomers possessing thermodynamic potential energies of -67.02 and -68.37 kcal/mole. There was only a small difference in the energy of the two isomers. There was no preferred isomer for the Re complex and both the *syn* and *anti* isomers were observed at an approximate ratio of 1:1. Molecular modeling of the Re complex of dimethylglycine-L-lysine-L-cysteine showed a difference between the thermodynamic potential energies of the two isomers to be approximately 1 kcal/mole. There was again only a small difference in the energy of the two isomers; hence, both the *syn* and *anti* isomers would be observed.

In the case of dimethylglycine-L-isoleucine-L-cysteine-glycine, a more bulky side chain was incorporated into the peptidic backbone. Molecular modeling of the Re complex of the dimethylglycine-L-isoleucine-L-cysteine-glycine showed one of the isomers having a potential energy that was approximately 3 kcal/mole lower than the energy of the other isomer. There was now a greater difference in the energies and there was a slight preference for one isomer over the other. Accordingly, the observed experimental ratio of the two isomers was 3:1.

In the case of dimethylglycine-L-t-butylglycine-L-cysteine-glycine, molecular modeling of the Re complex showed the difference in the potential energies of the two isomers to be approximately 6.5 kcal/mole. With the Re complex of dimethylglycine-D-t-butylglycine-L-cysteine-glycine, the difference in the energies of the two isomers was about 8.5 kcal/mole. One isomer was significantly preferred over the other; hence, only a single isomer was observed for the Re and Tc complexes.

Molecular modeling of the Re complex of mercaptoacetyl-L-t-butylglycine-glycine-glycine showed that the *syn* and anti isomers of the complex with a energy difference of 7.4. The metal complexes of mercaptoacetyl-L-t-butylglycine-glycine-glycine preferred one isomer over the other and would exist as a single isomer.

Artificial amino acids with bulky side chains can be prepared according to known literature methods.⁶³⁻⁶⁷ For example, both L- and D- amino acid derivatives can be prepared starting directly from the commercially available L- or D-serine, respectively.⁶⁷ Using this method, alkyl, phenyl and other bulky groups can be incorporated into serine to produce β-hydroxy-α-amino acids.⁶⁷ Hence, artificial amino acids with bulky side chains can be incorporated into peptidic chelators, which would produce a single species and a single isomeric metal complex.

The advantage of having a bifunctional chelator that forms a single isomeric metal complex is that in the labeling of biologically important molecules, there is only a single radiolabeled species. Hence, there is no need to isolate and evaluate the biological activity and toxicity of multiple compounds. It is also easier to formulate a radiopharmaceutical kit that consistently produces a single radiolabeled compound than one that produces a series of radiolabeled compounds. In the labeling of a biologically important molecule with a chelator that results in multiple species, there is a necessity to formulate the kit such that the labeling consistently produces the same set of compounds in the same ratio. This is eliminated with the use of a chelator that produces a single metal complex. Quality control of a radiopharmaceutical is also simplified by the use of a chelator that results in a single species as it is much easier to develop a quality control protocol that identifies a single well characterized compound than one that has to identify the presence and quantity of multiple compounds.

An additional benefit of the incorporation of different side chain groups into the peptidic chelator backbone to cause a single isomer is that the lipophilicity of the resulting metal complexes is altered by the addition of the different groups. The log D of the ^{99m}Tc complex of dimethylglycine-L-*t*-butylglycine-L-cysteine-glycine is -1.3, compared to -2.3 for the ^{99m}Tc complex of dimethylglycine-L-serine-L-cysteine-glycine.

The terms defining the variables R¹ - R¹⁰ , R^{a} - Rⁿ and X as used hereinabove in formula (I) have the following meanings:
"alkyl" refers to a straight or branched C₁-C₈ chain and includes lower C₁-C₄ alkyl;
"alkoxy" refers to straight or branched C₁-C₈ alkoxy and includes lower C₁-C₄ alkoxy;
"thiol" refers to a sulfhydryl group that may be substituted with an alkyl group to form a thioether;
"sulfur protecting group" refers to a chemical group that is bonded to a sulfur atom and inhibits oxidation of sulfur and includes groups that are cleaved upon chelation of the metal. Suitable sulfur protecting groups include known alkyl, aryl, acyl, alkanoyl, aryloyl, mercaptoacyl and organothio groups.

"Linking group" refers to a chemical group that serves to couple the targeting molecule to the chelator while not adversely affecting either the targeting function of the peptide or the metal binding function of the chelator. Suitable linking groups include alkyl chains; alkyl chains optionally substituted with one or more substituents and in which one or more carbon atoms are optionally replaced with nitrogen, oxygen or sulfur atoms. Other suitable linking groups include those having the formula A¹-A²-A³ wherein A¹ and A³ are independently selected from N, O and S; and A² includes alkyl optionally substituted with one or more substituents and in which one or more carbon atoms are optionally replaced with nitrogen, oxygen or sulfur atoms; aryl optionally substituted with one or more substituents; and heteroaryl optionally substituted with one or more substituents. Still other suitable linking groups include amino acids and amino acid chains functionalized with one or more reactive groups for coupling to the glycopeptide and/or chelator. In one embodiment, the linking group is a peptide of 1 to 5 amino acids and includes, for example, chains of 1 or more synthetic amino acid residues such as β-Alanine residues. In another embodiment, the linking group is NH-alkyl-NH.

"Targeting molecule" refers to a molecule that can selectively deliver a chelated radionuclide or MRI contrasting agent to a desired location in a mammal. Preferred targeting molecules selectively target cellular receptors, transport systems, enzymes, glycoproteins and processes such as fluid pooling. Examples of targeting molecules suitable for coupling to the chelator include, but are not limited to, steroids, proteins, peptides, antibodies, nucleotides and saccharides. Preferred targeting molecules include proteins and peptides, particularly those capable of binding with specificity to cell surface receptors characteristic of a particular pathology. For instance, disease states associated with over-expression of particular protein receptors can be imaged by labeling that protein or a receptor binding fragment thereof coupled to a chelator of invention. Most preferably targeting molecules are peptides capable of specifically binding to target sites and have three or more amino acid residues. The targeting moiety can be synthesised either on a solid support or in solution and is coupled to the next portion of the chelator-targeting moiety conjugates using known chemistry. peptides, antibodies, nucleotides and saccharides. Preferred targeting molecules include proteins and peptides, particularly those capable of binding with specificity to cell surface receptors characteristic of a particular pathology. For instance, disease states associated with over-expression of particular protein receptors can be imaged by labeling that protein or a receptor binding fragment thereof coupled to a chelator of invention. Most preferably targeting molecules are peptides capable of specifically binding to target sites and have three or more amino acid residues. The targeting moiety can be synthesised either on a solid support or in solution and is coupled to the next portion of the chelator-targeting moiety conjugates using known chemistry.

Chelator conjugates of the invention may be prepared by various methods depending upon the chelator chosen. The peptide portion of the conjugate if present is most conveniently prepared by techniques generally established in the art of peptide synthesis, such as the solid-phase approach. Solid-phase synthesis involves the stepwise addition of amino acid residues to a growing peptide chain that is linked to an insoluble support or matrix, such as polystyrene. The C-terminus residue of the peptide is first anchored to a commercially available support with its amino group protected with an N-protecting agent such as a t-butyloxycarbonyl group (tBoc) or a fluorenylmethoxycarbonyl (FMOC) group. The amino protecting group is removed with suitable deprotecting agents such as TFA in the case of tBOC or piperidine for FMOC and the next amino acid residue (in N-protected form) is added with a coupling agent such as dicyclocarbodiimide (DCC). Upon formation of a peptide bond, the reagents are washed from the support. After addition of the final residue, the peptide is cleaved from the support with a suitable reagent such as trifluoroacetic acid (TFA) or hydrogen fluoride (HF).

Conjugates may further incorporate a linking group component that serves to couple the peptide to the chelator while not adversely affecting either the targeting function of the peptide or the metal binding function of the chelator.

In accordance with one aspect of the invention, chelator conjugates incorporate a diagnostically useful metal capable of forming a complex, wherein the metal comprises the radionuclides technetium and rhenium in their various forms such as ^{99m}TcO³⁺,^{99m}TcO₂⁺, ReO³⁺ and ReO₂⁺. Incorporation of the metal within the conjugate can be achieved by various methods common in the art of co-ordination chemistry. When the metal is technetium-99m, the following general procedure may be used to form a technetium complex. A peptide-chelator conjugate solution is formed initially by dissolving the conjugate in aqueous alcohol such as ethanol. The solution is then degassed to remove oxygen then thiol protecting groups are removed with a suitable reagent, for example with sodium hydroxide and then neutralised with an organic acid such as acetic acid (pH 6.0-6.5). In the labelling step, a stoichiometric excess of sodium pertechnetate, obtained from a molybdenum generator is added to a solution of the conjugate with an amount of a reducing agent such as stannous chloride sufficient to reduce technetium and heated. The labelled conjugate may be separated from contaminants ^{99m}TcO₄ and colloidal ^{99m}TcO₂ chromatographically, for example with a C-18 Sep Pak cartridge.

In an alternative method, labelling can be accomplished by a transchelation reaction. The technetium source is a solution of technetium complexed with labile ligands facilitating ligand exchange with the selected chelator. Suitable ligands for transchelation include tartarate, citrate and heptagluconate. In this instance the preferred reducing reagent is sodium dithionite. It will be appreciated that the conjugate may be labelled using the techniques described above, or alternatively the chelator itself may be labelled and subsequently coupled to the peptide to form the conjugate; a process referred to as the "prelabelled ligand" method.

Another approach for labelling conjugates of the present invention involves techniques described in International Publication Number WO 95/13832. Briefly, the chelator conjugates are immobilized on a solid-phase support through a linkage that is cleaved upon metal chelation. This is achieved when the chelator is coupled to a functional group of the support by one of the complexing atoms. Preferably, a complexing sulfur atom is coupled to the support which is functionalized with a sulfur protecting group such as maleimide.

A conjugate labelled with a radionuclide metal such as technetium-99m may be administered to a mammal by intravenous injection in a pharmaceutically acceptable solution such as isotonic saline. The amount of labelled conjugate appropriate for administration is dependent upon the distribution profile of the chosen conjugate in the sense that a rapidly cleared conjugate may be administered in higher doses than one that clears less rapidly. Unit doses acceptable for imaging inflammation are in the range of about 5-40 mCi for a 70kg individual. In vivo distribution and localization is tracked by standard scintigraphic techniques at an appropriate time subsequent to administration; typically between 30 minutes and 180 minutes depending upon the rate of accumulation at the target site with respect to the rate of clearance at non-target tissue.

### List of Abbreviations

| **Abbreviation** | **Description** |
|---|---|
| Acm | acetoamidomethyl |
| Ar | argon |
| Arg | arginine |
| Boc | *tert*-butyloxycarbonyl |
| Cys | cysteine |
| DIEA | diisopropylethylamine |
| Dimethylgly | N,N-dimethylglycine |
| DMF | N,N-dimethylformamide |
| ES-MS | Electron Spray Mass Spectrometry |
| Fmoc | 9-fluorenylmethyloxycarbonyl |
| Gly | glycine |
| HBTU | 2-(1H-benzotriazol-1-yl)-1,1,3,3-tetramethyl-uronium |
| | hexafluorophosphate |
| HOBT | 1-hydroxybenzotriazole |
| HPLC | high performance liquid chromatography |
| Ile | isoleucine |
| Leu | leucine |
| Lys | lysine |
| mL | millilitre(s) |
| mmol | millimole(s) |
| mol | mole(s) |
| Mott | 4-methoxytrityl |
| NaOH | sodium hydroxide |
| NMP | N-methylpyrrolidone |
| Phe | phenylalanine |
| Pmc | 2,2,5,7,8-pentamethylchroman-6-sulfonyl |
| Rₜ | retention time |
| sasrin | 2-methoxy-4-alkoxybenzyl alcohol (super acid sensitive |
| resin) | |
| Ser | serine |
| *t*-Bu | *tert*-butyl |
| TFA | trifluoroacetic acid |
| Thr | threonine |
| Trt | trityl |
| Tyr | tyrosine |
| Y^{ε}-R | protection group R is attached to the peptide chain via the atom, |
| Y, | on the amino acid side chain (Y is N, O or S and R is |
| Acm, Boc, | Mott, *t*-Bu or Trt) |

### Examples

**Materials.** N-methylpyrrolidone, N,N-dimethylformamide, 100 mmol 2-(1H-benzotriazol-1-yl)-1,1,3,3-tetramethyl-uronium hexafluorophosphate/ 0.5M 1-hydroxybenzotriazole DMF, 2.0M diisopropylethylamine/ NMP, dichloromethane and trifluoroacetic acid were purchased from Applied Biosystems Inc. Triethylamine and *tert*-butyl methyl ether were purchased from Aldrich Chemical Inc. Fmoc amino acid derivatives and Fmoc-Gly sasrin resin was purchased from Bachem Bioscience Inc. All chemicals were used as received. [ReO₂(en)₂]Cl was prepared according to literature methods.^{57,58}

**Instrumentation.** NMR spectra were recorded on a Bruker AC-300 and on a Bruker DRX-500 NMR spectrometer and are reported as δ in ppm from external TMS. Mass spectra (electrospray) were obtained on a Sciex API#3 mass spectrometer in the positive ion detection mode. HPLC analyses and purifications were made on a Beckman System Nouveau Gold chromatographic system with a Waters 4 mm radial pak C-18 column. During analytical HPLC analysis, the mobile phase was changed from 100% 0.1% aqueous trifluoroacetic acid to 100% acetonitrile containing 0.1% trifluoroacetic acid over 20 minutes at a flow rate of 2 mL/min. All HPLC analyses were monitored with a UV detector set at 214 and 254 nm. Solid phase peptide syntheses were performed on an ABI Peptide Synthesizer model 433A using FastMoc chemistry and preloaded Fmoc amino acid sasrin resin.^{59,60} Molecular modeling of the Re complexes was performed using Quanta Charm version 3.3,⁶³ HPLC analyses of the ^{99m}Tc samples were made on a Beckman System Gold chromatographic system with a Vydac 4.6 mm radial pak C-18 column. The mobile phase was changed from 100% water containing 0.1% trifluoroacetic acid to 70% acetonitrile containing 0.1% trifluoroacetic acid over 25 minutes at a flow rate of 1 mL/min. The HPLC analyses of the ^{99m}Tc samples were monitored with a UV detector set at 215 nm and a radiometric gamma detector.

### Example 1

**Synthesis of Peptides**. Peptides of various amino acid sequences were prepared via a solid phase peptide synthesis method on an automated peptide synthesizer using FastMoc 1.0 mmole chemistry.^{59,60} Preloaded Fmoc amino acid sasrin resin and Fmoc amino acid derivatives were used. Prior to the addition of each amino acid residue to the N-terminus of the peptide chain, the FMOC group was removed with 20% piperidine in NMP. Each Fmoc amino acid residue was activated with 0.50 M HBTU/ HOBt/ DMF, in the presence of 2.0M DIEA/ NMP. The C-terminus of the completed peptide was attached to the resin via the sasrin linker. The peptidyl resin was washed with dichloromethane and dried under vacuum for 20-24 hours. The peptide was cleaved off the resin by stirring the peptidyl resin in 95 % aqueous trifluoroacetic acid for 3-4 hours. The sasrin resin was filtered and the filtrate was added dropwise to *tert-*butyl methyl ether at 0 °C. The peptide precipitate out of the ether. The precipitate was collected by centrifugation and dissolved in minimal amount of water. The aqueous peptide solution was lyophilized to yield the product. The product was analyzed by mass spectrometry and by HPLC. The product was purified by HPLC. This method was used to prepare the following peptides
1)RP349: Dimethylgly-L-Ile-L-Cys(S^{ε}-Acm)-Gly
2)RP332: Dimethylgly-L-lysine(N^{ε}-Biotin)-L- Cys(S^{ε}-Acm)
3)RP455: Dimethylgly-L-*t*-Butylgly-L-Cys(S^{ε}-Acm)-Gly
4)RP505: Dimethylgly-D-*t*-Butylgly-L-Cys(S^{ε}-Acm)-Gly
5)RP502: Dimethylgly-L-*t*-Butylgly-L-Cys(S^{ε}-Acm)-Gly-Thr-Lys-Pro-Pro-Arg
6)RP573: Dimethylgly-L-*t*-Butylgly-L-Cys(S^{ε}-Acm)-Gly-Arg-Ile-Lys-Pro-His

### Example 2

**Synthesis of Re Oxo Complex of Dimethylglycine-L-*t-*butylgly-L-Cys-Gly:** To remove the acm protecting group, dimethylgly-L-*t*-butylgly-L-Cys-(S^{ε}-Acm)-Gly (84.0 mg, 0.187 mmoles) was dissolved in 2 mL of 30% acetic acid. Mercury(II) acetate (91.6 mg, 0.287 mmoles) was added to the solution and the solution was stirred under Ar at room temperature for 18 hours. H₂S gas was then bubbled through the solution for 5 minutes, causing black HgS to precipitate. The precipitate was removed by centrifugation, and the filtrate was frozen and lyophilized overnight. [ReO₂(en)₂]Cl (88.6 mg, 0.237 mmoles) was dissolved in 3 mL of distilled water and added to the lyophilized deprotected peptide. The solutions was a light green colour. The pH of the solution was adjusted to 6 using 1 M NaOH. The solution was refluxed under Ar for 2 hours, during which time the solution changed from green to red. The solution was frozen and lyophilized overnight, yielding a red solid. Purification of the product was done by HPLC. Mass spectrum (electrospray): *m*/*z* = 577 ([MH]⁺), [C₁₅H₂₇NaO₆Re₁S₁]. HPLC retention time: 9.52 min. ¹H NMR and ¹³C NMR (500 MHz, D₂O) spectral data are shown in Table 3 and 4. Log D (pH: 7.4): -1.3.

### Example 3

**Synthesis of Re Oxo Complex** of **Dimethylgly-D-*t-*butylgly-L-Cys-Gly:** The procedure for the synthesis of the Re oxo complex of dimethylgly-D-*t*-butylgly-L-Cys-Gly was the same as the one described for the synthesis of the Re complex of Dimethylgly-L-*t*-butylgly-L-Cys-Gly. Mass spectrum (electrospray): *m*/*z* = 577 ([MH⁺), [C₁₅H₂₆N₄O₆Re₁S₁]. HPLC retention time: 9.62 min. ¹H NMR (300 MHz, D₂O): 2.89 (s, methyl ¹H in the dimethylglycine residue), 3.65 (s, methyl ¹H in the dimethylglycine residue).

### Example 4

**Synthesis of Re Oxo Complex of Dimethylgly-L-*t*-Butylgly-L-Cys-Gly-Thr-Lys-Pro-Pro-Arg:** The procedure for the synthesis of the Re oxo complex Dimethylgly-L-*t*-Butylgly-L-Cys-Gly-Thr-Lys-Pro-Pro-Arg was the same as the one described for the synthesis of the Re complex of dimethylgly-L-*t*-butylgly-L-Cys-Gly. Mass spectrum (electrospray): *m*/*z* = 1155 ([MH]⁺), [C₄₁H₇₁N₁₃O₁₂Re₁S₁]⁺). HPLC retention time: 8.82 min. ¹H NMR (500 MHz, D₂O): 2.63 (s, methyl ¹H in the dimethylglycine residue), 3.56 (s, methyl ¹H in the dimethylglycine residue).

### Example 5

**Synthesis of Re Oxo Complex of Dimethylgly-L-Ile-L-Cys-Gly:** The procedure for the synthesis of the Re oxo complex Dimethylgly-L-ile-L-cys-gly was the same as the one described for the synthesis of the Re complex of dimethylgly-L-*t*-butylgly-L-cys-gly. Mass spectrum (electrospray): *m*/*z* = 577 ([MH]⁺, [C₄₁H₇₁N₁₃O₁₂Re₁S₁]⁺), *m*/*z* = 598 ([MH]⁺, [C₄₁H₇₁N₁₃O₁₂Re₁S₁]⁺). HPLC retention time: 9.50 min. ¹H NMR (300 MHz, D₂O): 2.60 (s, methyl ¹H in the dimethylglycine residue of isomer A), 2.76 (s, methyl ¹H in the dimethylglycine residue of isomer B), 3.68 (s, methyl ¹H in the dimethylglycine residue of isomer A), 3.72 (s, methyl ¹H in the dimethylglycine residue of isomer B).

### Example 6

**Synthesis of the Re Oxo Complex of Dimethylgly-L-*t*-Butylgly-Cys**-**Gly-Arg-Ile-Lys-Pro-His:** The procedure for the synthesis of the Re oxo complex of dimethylgly-L-*t*-Butylgly-L-Cys-Gly-Arg-Ile-Lys-Pro-His was the same as the one described for the synthesis of the Re complex of dimethylgly-L-*t*-butylgly-L-Cys-Gly. Mass spectrum (electrospray): *m*/*z* = 1207 ([MH]⁺, [C₄₃H₇₁N₁₅O₁₀Re₁S₁]. HPLC retention time: 8.78 min. ¹H NMR (300 MHz, D₂O): 2.71 (s, methyl ¹H in the dimethylglycine residue), 3.65 (s, methyl ¹H in the dimethylglycine residue).

### Example 7

**Synthesis of the ^{99m}Tc complex.** The peptide (0.2-0.5 µmoles) was dissolved in 200 µL of saline. Na[^{99m}TcO₄] (10 mCi) was added to the solution , followed by tin(II) chloride (7.5 x 10³ µg, 39 µmoles), sodium gluconate (1.3 x 10³ µg, 5.8 µmoles), and 20 µ**L** of 0.1 M NaOH. The solution was left at room temperature for 1 hour or heated at 100°C for 15 minutes. In the synthesis of the ^{99m}Tc complex, the acetoamidomethyl protection group was displaced from the cysteine residue in RP414. The ^{99m}Tc complex was analyzed by HPLC. The ^{99m}Tc complexes of RP455, RP505 and RP502 was co-injected with the corresponding Re complexes. The HPLC retention times of the ^{99m}Tc peptidic complexes are as follows:
1)^{99m}Tc complex of RP349 (Dimethylgly-L-Ile-L-Cys-Gly): HPLC retention time: ^{99m}Tc(RP349) Rₜ = 19.41, 21.53 min (radiometric gamma detector).
2)^{99m}Tc complex of RP332 (Dimethylgly-L-lysine(N^{ε}-Biotin)-L- Cys): HPLC retention time: ^{99m}Tc(RP332) Rₜ = 11.54, 11.97 min (radiometric gamma detector).
3)^{99m}Tc complex of RP455 (Dimethylgly-L-*t*-Butylgly-L-Cys-Gly): HPLC retention time: ReO(RP455) Rₜ = 21.18 min (UV detector set at 215 nm); ^{99m}Tc(RP445) Rₜ = 21.49 min (radiometric gamma detector).
4) ^{99m}Tc complex of RP505 (Dimethylgly-D-*t*-Butylgly-L-Cys-Gly): HPLC retention time: ReO(RP505) Rₜ = 18.16 min (UV detector set at 215 nm); ^{99m}Tc(RP505) Rₜ = 18.89 min (radiometric gamma detector).
5)^{99m}Tc complex of RP502 (Dimethylgly-L-*t*-Butylgly-L-Cys(S^{ε}-Acm)-Gly-Thr-Lys-Pro-Pro-Arg): HPLC retention time: ReO(RP502) Rₜ = 19.76 min (UV detector set at 215 nm); ^{99m}Tc(RP502) Rₜ = 20.10 min (radiometric gamma detector).
6) ^{99m}Tc complex of RP573 (Dimethylgly-L-*t*-Butylgly-L-Cys(S^{ε}-Acm)-Gly-Arg-Ile-Lys-Pro-His): HPLC retention time: (ReORP573) Rₜ = 16.43 min (UV detector set at 215 nm); ^{99m}Tc(RP573) Rt = 20.75 min (radiometric gamma detector).

### Example 8

**Synthesis of Dimethylgly-L-Beta-hydroxyvaline-L-Cys-Gly.** The beta-hydroxyvaline is synthesized according to the method of Shao, H., and Goodman, M., J. Org. Chem. 1996, 61, 2582-2583 or Beloken, Yu. N.; Bulychev, A. G.; Vitt, S. V.; Struchkov, Yu. T.; Batsanov, A. S.; Timofeeva, T. V.; Tsyryapkin, V. A.; Ryzhov, M. G.; Lysova, L. A.; Et al. J. Am. Soc. Chem., 1985, 107(14), 4252-9.. The FMOC group is added to the amino terminus according to the method of Carpino, L. A., Han, G. Y. J. Org. Chem. 1972, 37, 3404. The FMOC-beta-hydroxyvaline is purified by column chromatography. The peptide dimethylgly-L-beta-hydroxyvaline-L-cys-gly is synthesized on the peptide synthesizer in the same manner as set out in Example 1. The Re and Tc-99m complexes are synthesized by the same method as the Re and Tc-99m complexes of dimethylgly-L-t-butylgly-L-cys-gly as shown in examples 2 and 7 respectively.

These Re and Tc-99m chelates form the syn isomer predominantly but are more hydrophilic than the Re and Tc-99m complexes already mentioned. This is an advantage when the chelates are attached to hydrophilic targeting molecules.

Although the invention has been described with preferred embodiments, it is to be understood that modifications may be resorted to as will be apparent to those skilled in the art. Such modifications and variations are to be considered within the purview and scope of the present invention.

### References

(1) Baidoo, K. E.; Lever, S. Z. Bioconjugate Chem. 1990, 1, 132.
(2) Eisenhut M.; Missfeldt, M.; Lehmann, W. D.; Karas, M. J. Label Compound Radiopharm. 1991, 29, 1283.
(3) Fritzberg, A. R.; Beaumier, P. L. J. Nucl. Med. 1992, 33, 394.
(4) Fischman, A. J.; Babich, J. W.; Strauss, H. W. J. Nucl. Med. 1993, 34, 2253.
(5) Thakur, M. L. Nucl. Med. Commun. 1995, 16, 724.
(6) Malin, R.; Steinbrecher, R.; Jannsen, J.; Semmler, W.; Noll, B.; Johannsen, B.; Frommel, C.; Hohne, W.; Schneider-mergener, J. J. Am. Chem. Soc. 1995, 117, 11821.
(7) Pearson, D. A.; Lister-James, J.; McBride, W. J.; Wilson, D. M.; Martel, L. J.; Civitello, E. R.; Taylor, J. E.; Moyer, B. R.; Dean, R. T. J. Med. Chem. 1996, 39, 1361.
(8) Lister-James, J.; Knight, L. C.; Maurer, A. H.; Bush, L. R. J. Nucl. Med. 1996, 37, 775.
(9) Liu, S.; Edwards, D. S.; Looby, R. J.; Harris, A. R.; Poirier, M. J.; Barrett, J. A.; Heminway, S. J.; Carroll, T. R. Bioconjugate Chem. 1996, 7, 63.
(10) Liu, S.; Edwards, D. S.; Looby, R. J.; Poirier, M. J.; Rajopadhye, M.; Bourque, J. P.; Carroll, T. R. Bioconjugate Chem. 1996, 7, 196.
(11) Deutsch, E.; Libson, K.; Jurisson, S.; Lindoy, L. F. Prog. Inorg. Chem. 1983, 30, 75.
(12) Melnik, M.; Van Lier, J. E. Coord Chem. Rev. 1987, 77, 275.
(13) Mazzi, U. Polyhedron, 1989, 8, 1633.
(14) Jurisson, S.; Bering, D.; Jia, W.; Ma, D. Chem. Rev. 1993, 93, 1137.
(15) Tisato, F.; Refosco, F.; Bandoli, G. Coord. Chem. Rev. 1994, 135, 325.
(16) Otsuka, F. L.; Welch, M. J. Nucl. Med. Biol. 1987, 14, 243.
(17) Fritzberg, A. R.; Berninger, R. W.; Hardey, S. W.; Wester, D. W. Pharm. Res. 1988, 5, 325.
(18) Eckelman, W. C.; Paik, C. H.; Steigman, J. Nucl. Med. Biol. 1989, 16, 171.
(19) Hnatowich, D. J. Semin. Nucl. Med. 1990, 20, 80.
(20) Srivastava, s. C.; Mease, R. C. Nucl. Med. Biol. 1991, 18, 589.
(21) Liang, F. H.; Virzi, F.; Hnatowich, D. J. Nucl. Med Biol. 1987, 14, 63.
(22) Chianelli, M.; Signore, A.; Fritzberg, A. R.; Mather, S. J. Eur. J. Nucl. Med. 1992, 19, 625.
(23) Baidoo, K. E.; Lever, S. Z.; Scheffel, U. Bioconjugate Chem. 1994, 5, 114.
(24) Eisenhut, M.; Lehmann, W. D.; Becker, W.; Behr, T. J. Nucl. Med. 1996, 37, 362.
(25) Edwards, D. S.; Liu, S.; Barrett, J. A.; Harris, A. R; Looby, R. J.; Ziegler, M. C.; Heminway, S. J.; Carroll, T. R. Bioconjugate Chem. 1997, 8, 146.
(26) Barrett, J. A.; Crocker, A. C.; Damphousse, D. J.; Heminway, S. J.; Liu, S.; Edwards, D. S.; Lazewatsky, J. L.; Kagun, M.; Mazaika, T. J.; Carroll, T. R. Bioconjugate Chem. 1997, 8, 155.
(27) Thakur, M. L.; Kolan, H.; Li, J.; Wiaderkiewicz, R.; Pallela, V. R.; Duggaraju, R.; Schally, A. V. Nucl. Med. Biol. 1997, 24, 105.
(28) Childs, R. L.; Hnatowich, D. J. J. Nucl. Med. 1985, 26, 293.
(29) Fischman, A. J.; Babich, J. W.; Rubin, H. R. Semin. Nucl. Med. 1993, 24, 1954.
(30) Babich, J. W.; Solomon, H.; Pike, M. C.; Kroon, D.; Graham, W.; Abrams, M. J.; Tompkins, R. G.; Rubin, R. H.; Fischman, A. J. J. Nucl. Med. 1993, 34, 1967.
(31) Babich, J. W.; Fichman, A. J. Nucl. Med. Biol. 1995, 22, 25.
(32) Hosotani, T.; Yokoyama, A.; Arano, Y.; Horiuchi, K.; Wasaki, H.; Saji, H.; Torizuka, K. Nucl. Med. Biol. 1986, 12, 431.
(33) Leonard, J. P.; Nowotnik, D. P.; Neirinckx, R. D. J. Nucl. Med. 1986, 27, 1819.
(34) Neirinckx, R. D.; Canning, L. R.; Piper, I. M.; Nowotnik, d P.; Pickett, R. D.; Holmes, R. A.; Volkert, W. A.; Forster, A. M.; Weisner, P. S.; Marriott, J. A.; Chaplin, S. B. J. Nucl. Med. 1987, 28, 191.
(35) Neirinckx, R. D.; Burke, J. F.; Harrison, R. C.; Forster, A. M.; Andersen, A. R.; Lassen, N. A. J. Cereb. Blood Flow Metab. 1988, 8, S4.
(36) Holm, S.; Anderson, A. R.; Vorstrup, S.; Lassen, N. A.; Paulson, O. B.; Holmes, R. A.; J. Nucl. Med. 1985, 26, 1129.
(37) Sharp, P. F.; Smith, F. W.; Gemmell, H. G.; Lyall, D.; Evans, N. T. S.; Gvozdanovic, D.; Davidson, J.; Tyrrell, D. A.; Pickett, R. D.; Neirinckx, R. D. J. Nucl. Med. 1986, 27, 171
(38) Linder, K. E.; Wen, M. D.; Nowotnik, D. P.; Malley, M. F.; Gougoutas, J. Z.; Nunn, A. D.; Eckelman, W. C. Bioconjugate Chem. 1991, 2, 160
(39) Rao, T. N.; Adhikesavalu, D.; Camerman, A.; Fritzberg, A. R. J. Am. Chem. Soc. 1990, 112, 5798
(40) Eshima, D.; Taylor Jr., A.; Fritzberg, A. R.; Kasina, S.; Hansen, L.; Sorenson, J. F. J. Nucl. Med. 1987, 28, 1180
(41) Subhani, M.; Cleynhens, B.; Bormans, G.; Hoogmartens, M.; De Roo, M.; Verbruggen, A. M. In Technetium and Rhenium in Chemistry and Nuclear Medicine-3; Nicoline M.; Banoli, G.; Mazzi, U., Eds.; Cortina International, Verona, Italy, 1990, p. 453.
(42) Bormans, G.; Cleynhens, B.; Hoogmartens, M.; De Roo, M.; Verbruggen, A. M. In Technetium and Rhenium in Chemistry and Nuclear Medicine-3; Nicoline M.; Banoli, G.; Mazzi, U., Eds.; Cortina International, Verona, Italy, 1989, p. 661.
(43) Bormans, G.; Cleynhens, B.; Adriaens, P.; De Roo, M.; Verbruggen, A. M. J. Labelled Compounds and Radiopharmaceuticals, 1993, 33, 1065
(44) Lister-James, J.; Knight, L. C.; Mauer, A. H.; Bush, L. R.; Moyer, B. R.; Dean, R. T. J. Nucl. Med. 1996, 37, 775
(45) Muto, P.; Lastoria, S.; Varrella, E.; Salvatore, M.; Morgano, G.; Lister-James, J.; Bemardy, J. D.; Dean, R. T. Wencker, D.; Boer, J. S. J. Nucl. Med. 1995, 36, 1384
(46) Klingensmith III, W. C.; Fritzberg, A. R.; Spitzer, V. M.; Johnson, D. L.; Kuni, C. C.; Williamson, M. R.; Washer, G.; Weil III, R. J. Nucl. Med. 1984, 25, 42.
(47) Marchi, A.; Marvelli, L.; Rossi, R.; Magon, L.; Bertolasi, V.; Ferretti, V.; Gilli, P.; J. Chem. Soc., Dalton Trans. 1992, 1485
(48) Kung, H. F.; Bradshaw, J. E.; Chumpradit, S.; Zhang, Z. P.; Kung, M. P.; Mu, M.; Frederick, D. In Technetium and Rhenium in Chemistry and Nuclear Medicine-4; Nicoline M.; Banoli, G.; Mazzi, U., Eds.; Cortina International, Verona, Italy, 1995, p. 293.
(49) Meegalla, S.; Plossl, K.; Dung, M.-P.; Chumpradt, S.; Stevenson, D. A.; Kushner, S. A.; McElgin, W. T.; Mozley, P. D.; Kung, H. F. J. Med. Chem. 1997, 40, 9
(50) Edwards, D. S.; Cheesman, E. H.;; Watson, M. W.; Maheu, L. J.; Nguyen, S. A.; Dimitre, L.; Nason, T.; Watson, A. D.; Walovitch, R In Technetium and Rhenium in Chemistry and Nuclear Medicine-3; Nicoline M.; Banoli, G.; Mazzi, U., Eds.; Cortina International, Verona, Italy, 1990, p. 431.
(51) Oya, S.; Kung, M.-P.; Frederick, D.; Kung, H. F. Nucl. Med. Biol. 1995, 22, 749.
(52) Kung, H. F.; Guo, Y. Z.; Yu, C. C.; Billings, J.; Subramanyam, B.; Calabrese, J. C. J. Med. Chem. 1989, 32, 433.
(53) Mach, R. H.; Kung, H. F.; Guo, Y. Z.; Yu, C. C.; Subramanyam, V.; Calabrese, J. C. Nucl. Med. Biol. 1989, 16, 829.
(54) Francesconi, L. C.; Graczyk, G.; Wehrli, S.; Shaikh, S. N.; McClinton, D.; Liu, S.; Zubieta, J.; Kung, H. F. Inorg. Chem. 1993, 32, 3114.
(55) Efange, S. M. N.; Kung, H. F.; Billings, S. S.; Blau, M. J. Med. Chem. 1988, 31, 1043.
(56) Walovitch, R. C.; Cheesman, E. H.; Maheu, L. J.; Hall, K. M. J. Cereb. Blood Flow Metab. 1988, 8, S4.
(57) Rouschias, G. Chem. Rev. 1974, 74, 531.
(58) Fergusson, J. E. Coord. Chem. Rev. 1966, 1, 459.
(59) User' Manual of Peptide Synthesizer Model 433A, Applied BioSystems, Philadelphia, 1993.
(60) Introduction to Cleavage Techniques, Applied BioSystems, Philadelphia, 1990.
(61) Wong, E.; Fauconnier, T.; Bennett, S.; Valliant J.; Nguyen, T.; Lau, F.; Lu, L. F. L.; Pollak,; Bell, R. A.; Thornback, J. R. Inorg. Chem. 1997, in press.
(62) Peers, S. H.; Tran, L. L.; Eriksson, S. J.; Ballinger, J.; Goodbody, A. E. J. Nucl. Med. 1995, 36, 114P.
(63) Williams, R. M. Synthesis of Optically Active a-Amino Acids; Pergamon: Toronto, Canca, 1987.
(64) Arnold, L. D.; May, r. G.; Vederas, J. C. J. Am. Chem. Soc. 1987, 109, 4649.
(65) Arnold, L. D.; May, R. G.; Vederas, J. C. J. Am. Chem. Soc. 1988, 110, 2237.
(66) Reetz, M. T. Angew. Chem., Int. Ed. Engl. 1991, 30, 1531.
(67) Blaskovich, M. A.; Lajoie, G. A. J. Am. Chem. Soc. 1993, 115, 5021.
(68) Shao, H., and Goodman, M., J. Org. Chem. 1996, 61, 2582-2583.
(69) Beloken, Yu. N.; Bulychev, A. G.; Vitt, S. V.; Struchkov, Yu. T.; Batsanov, S.; Timofeeva, T. V.; Tsyryapkin, V.A.; Ryzhov, M. G.; Lysova, L. A.; Et al. J. Am. Soc. Chem., 1985, 107(14), 4252-9.
(70) Carpino, L. A., Han, G. Y. J. Org. Chem. 1972, 37, 3404.

## Claims

1. A compound that predominately forms a single stereoisomer upon coordination to a metal center of the following formula 1: 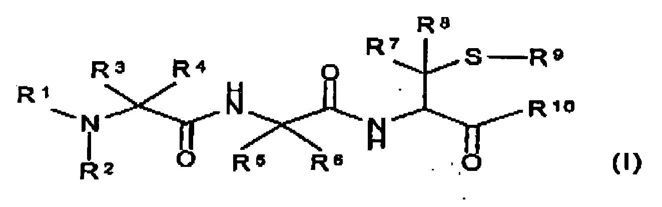 wherein
R¹ is a linear or branched, saturated or unsaturated C₁₋₄alkyl chain that is optionally interrupted by one or two heteroatoms selected from N, O and S; and is optionally substituted by one or more substituents selected from halogen, hydroxyl, amino, carboxyl, C₁₋₄alkyl, aryl, and C(O)R¹⁰;
R² is H or a substituent defined by R¹;
R¹ and R² may together form a 5- to 8-membered saturated or unsaturated heterocyclic ring optionally substituted by one or more substituents selected from halogen, hydroxyl, amino, carboxyl, oxo, C₁₋₄alkyl and aryl;
R³, R⁴ and R⁵ are selected independently from H; carboxyl; C₁₋₄alkyl; C₁₋₄alkyl substituted with a substituent selected from hydroxyl, amino, sulfhydryl, halogen, carboxyl, C₁₋₄alkoxycarbonyl and aminocarbonyl; an alpha carbon side chain of a D- or L-amino acid other than proline; and C(O)R¹⁰;
R⁶ is selected from the group consisting of:
(i) an optionally substituted 3- to 6-membered heterocylic or carbocylic ring;
(ii) a compound having the following formula: 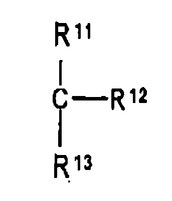 wherein R¹¹, R¹² and R¹³ are independently selected from H, linear or branched, saturated or unsaturated C₁₋₆alkyl chain that is optionally interrupted by one or two heteroatoms selected from N, O and S; and is optionally substituted by one or more substituents selected from alkoxycarbonyl, aminocarbonyl, alkoxy, an optionally substituted 3- to 6- membered heterocylic or carbocylic ring; with the proviso that a least one of R¹¹, R¹² and R¹³ is not H;
(iii) a compound of the following formula: 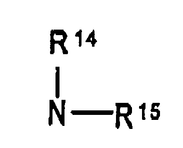 wherein R¹⁴ and R¹⁵ are independently selected from H, linear or branched, saturated or unsaturated C₁₋₆alkyl chain that is optionally interrupted by one or two heteroatoms selected from N, O and S; and is optionally substituted by one or more substituents; alkoxycarbonyl, aminocarbonyl, alkoxy, an optionally substituted 3- to 6-membered heterocylic or carbocylic ring; with the proviso that a least one of R¹⁴ and R¹⁵ is not H; and
(iv) a compound of the following formula: 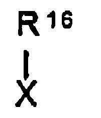
wherein X is selected from O or S and R¹⁶ is selected from linear or branched, saturated or unsaturated C₁₋₆alkyl chain that is optionally interrupted by one or two heteroatoms selected from N, O and S; and is optionally substituted by one or more substituents selected from alkoxycarbonyl, aminocarbonyl, alkoxy, and an optionally substituted 3- to 6-membered heterocylic or carbocylic ring;
R⁷ and R⁸ are selected independently from H; carboxyl; amino; C₁₋₄alkyl; C₁₋₄alkyl substituted by a substituent selected from hydroxyl, carboxyl and amino; and C(O)R¹⁰;
R⁹ is selected from H and a sulfur protecting group;
R¹⁰ is selected from hydroxyl, alkoxy, an amino acid residue, a linking group and a targeting molecule; and
wherein the metal center is selected from technetium or rhenium.

2. A compound that predominately forms a single stereoisomer upon coordination to a metal center of the following formula II: 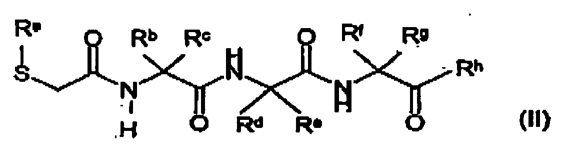 wherein
R^{a} is selected from H and a sulfur protecting group;
R^{b}, R^{c} R^{d}, R^{f} and R^{g} are selected independently from H; carboxyl; C₁₋₄alkyl C₁₋₄alkyl substituted with a substituent selected from hydroxyl, amino, sulfhydryl, halogen, carboxyl, C₁₋₄ alkoxycarbonyl and aminocarbonyl; an alpha carbon side chain of a D- or L-amino acid other than proline; and C(O)R^{h};
R^{e} is an optionally substituted 3- to 6-membered heterocylic or carbocylic ring;
or R^{e} is 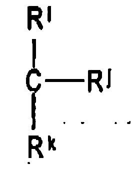 wherein Rⁱ, R^{j} and R^{k} are independently selected from H, linear or branched, saturated or unsaturated C₁₋₆alkyl chain that is optionally interrupted by one or two heteroatoms selected from N, O and S; and is optionally substituted by one or more substituents; alkoxycarbonyl, aminocarbonyl, alkoxy, an optionally substituted 3- to 6-membered heterocylic or carbocylic ring; with the proviso that a least one of Rⁱ, R^{j} and R^{k} is not H;
or R^{e} is 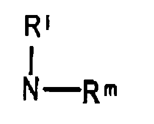 wherein R^{l} and R^{m} are independently selected from H, linear or branched, saturated or unsaturated C₁₋₆alkyl chain that is optionally interrupted by one or two heteroatoms selected from N, O and S; and is optionally substituted by one or more substituents; alkoxycarbonyl, aminocarbonyl, alkoxy, an optionally substituted 3- to 6-membered heterocylic or carbocylic ring; with the proviso that at least one of R^{l} and R^{m} is not H;
or R^{e} is 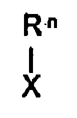 wherein X is selected from O or S and Rⁿ is selected from linear or branched, saturated or unsaturated C₁₋₆alkyl chain that is optionally interrupted by one or two heteroatoms selected from N, O and S; and is optionally substituted by one or more substituents; alkoxycarbonyl, aminocarbonyl, alkoxy, and an optionally substituted 3- to 6-membered heterocylic or carbocylic ring;
R^{h} is selected from hydroxyl, alkoxy, an amino acid residue, a linking group and a targeting molecule; and
wherein the metal center is selected from technetium or rhenium.

3. A compound that predominately forms a single stereoisomer upon coordination to a metal center selected from:
Dimethylgly-L-t-Butylgly-L-Cys-Gly;
Dimethylgly-D-*t*-Hutylgly-L-Cys-Gly;
Dimethylgly-L-*t*-Butylgly-L-Cys;
Dimethylgly-L-*t*-Butylgly-L-Cys(S^{c}-Acm)-Gly-Thr-Lys-Pro-Pro-Arg;
wherein the metal center is selected from technetium or rhenium.

4. A compound according to any of claims 1 to 3 in a form complexed with a metal or metal radionuclide or an oxide or nitride thereof, wherein the mental is selected from technetium or rhenium.

5. A pharmaceutical composition, comprising a pharmaceutically acceptable carrier and a compound as defined in claim 4 in an amount effective to image a site of diagnostic interest.

6. A pharmaceutical composition as defined in claim 5 for use in radioimaging a site of diagnostic interest.

## Patentansprüche

1. Eine Verbindung, welche nach Koordination an ein Metallzentrum überwiegend ein einzelnes Stereoisomer der folgenden Formel I bildet: 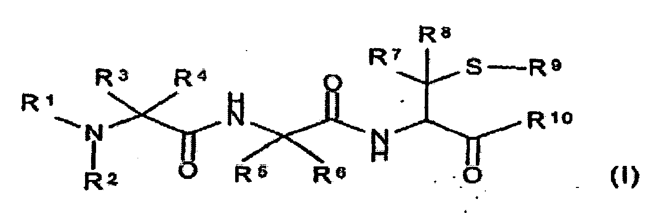 wobei
R¹ eine lineare oder verzweigte, gesättigte oder ungesättigte C₁₋₄Alkyl-Kette ist, die wahlweise durch ein oder zwei Heteroatome ausgewählt aus N, O und S unterbrochen ist; und wahlweise durch einen oder mehrere Substituenten ausgewählt aus Halogen, Hydroxyl, Amino, Carboxyl, C₁₋₄Alkyl, Aryl und C(O)R¹⁰ substituiert ist;
R² H oder ein durch R¹ definierter Substituent ist;
R¹ und R² gemeinsam einen 5- bis 8-gliedrigen gesättigten oder ungesättigten heterocyclischen Ring bilden können, der wahlweise durch einen oder mehrere Substituenten ausgewählt aus Halogen, Hydroxyl, Amino, Carboxyl, Oxo, C₁₋₄Alkyl und Aryl substituiert ist;
R³, R⁴ und R⁵ unabhängig von H; Carboxyl; C₁₋₄Alkyl ausgewählt sind;
C₁₋₄Alkyl mit einem Substituenten substituiert ist ausgewählt aus Hydroxyl, Amino, Sulfhydryl, Halogen, Carboxyl,
C₁₋₄Alkoxycarbonyl und Aminocarbonyl; einer Alpha-Kohlenstoff-Seitenkette von einer D- oder L-Aminosäure, die kein Prolin ist; und C(O)R¹⁰_{;}
R⁶ ausgewählt ist aus der Gruppe bestehend aus:
(i) einem wahlweise substituierten 3- bis 6-gliedrigen heterocyclischen oder carbocyclischen Ring;
(ii) einer Verbindung mit der folgenden Formel: 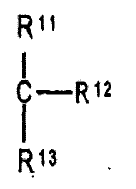 wobei R¹¹, R¹² und R¹³ unabhängig ausgewählt sind von H, einer linearen oder verzweigten, gesättigten oder ungesättigten C₁₋₆Alkyl-Kette, die wahlweise durch ein oder zwei Heteroatome ausgewählt aus N, O und S unterbrochen ist; und die wahlweise durch einen oder mehrere Substituenten substituiert ist ausgewählt aus Alkoxycarbonyl, Aminocarbonyl, Alkoxy, einem wahlweise substituierten 3- bis 6-gliedrigen heterocyclischen oder carbocyclischen Ring; mit der Maßgabe, dass zumindest eines von R¹¹, R¹² und R¹³ nicht H ist;
(iii) einer Verbindung der folgenden Formel: 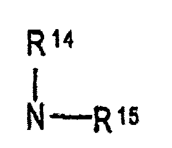 wobei R¹⁴ und R¹⁵ unabhängig ausgewählt sind von H, einer linearen oder verzweigten, gesättigten oder ungesättigten C₁₋₆Alkyl-Kette, die wahlweise durch ein oder zwei Heteroatome ausgewählt aus N, O und S unterbrochen ist; und die wahlweise substituiert ist durch einen oder mehrere Substituenten; Alkoxycarbonyl, Aminocarbonyl, Alkoxy, einen wahlweise substituierten 3- bis 6-gliedrigen heterocyclischen oder carbocyclischen Ring; mit der Maßgabe, dass zumindest eines von R¹⁴ und R¹⁵ nicht H ist; und
(iv) einer Verbindung der folgenden Formel: 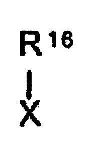
wobei X aus O oder S ausgewählt und R¹⁶ aus einer linearen oder verzweigten, gesättigten oder ungesättigten C₁₋₆AlkylKette ausgewählt ist , die wahlweise durch ein oder zwei Heteroatome ausgewählt aus N, O und S unterbrochen ist; und wahlweise durch einen oder mehrere Substituenten ausgewählt aus Alkoxycarbonyl, Aminocarbonyl, Alkoxy, und einem wahlweise substituierten 3- bis 6-gliedrigen heterocyclischen oder carbocyclischen Ring substituiert ist;
R⁷ und R⁸ unabhängig ausgewählt sind aus H; Carboxyl; Amino; C₁₋₄Alkyl; C₁₋₄Alkyl, das durch einen Substituenten substituiert ist ausgewählt aus Hydroxyl, Carboxyl und Amino; und C(O)R¹⁰;
R⁹ aus H und einer Schwefel-Schutzgruppe ausgewählt ist;
R¹⁰ aus Hydroxyl, Alkoxy, einem Aminosäurerest, einer Verbindungsgruppe und einem Targetingmolekül ausgewählt ist; und
wobei das Metallzentrum aus Technetium oder Rhenium ausgewählt ist.

2. Eine Verbindung, welche nach Koordination an ein Metallzentrum überwiegend ein einzelnes Stereoisomer der folgenden Formel II bildet: 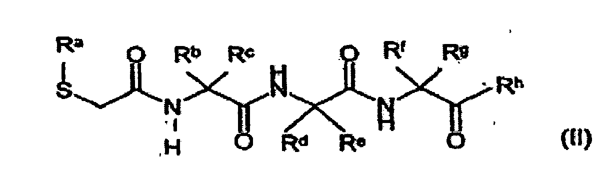 wobei
R^{a} aus H und einer Schwefel-Schutzgruppe ausgewählt ist;
R^{b}, R^{c}, R^{d}, R^{f} und R^{g} unabhängig ausgewählt sind aus H; Carboxyl; C₁₋₄Alkyl; C₁₋₄Alkyl, das mit einem Substituenten substituiert ist ausgewählt aus Hydroxyl, Amino, Sulfhydryl, Halogen, Carboxyl, C₁₋₄Alkoxycarbonyl und Aminocarbonyl; einer Alpha-Kohlenstoff-Seitenkette von einer D- oder L-Aminosäure, die kein Prolin ist; und C(O)R^{h};
R^{e} ein wahlweise substituierter 3- bis 6-gliedriger heterocyclischer oder carbocyclischer Ring ist; oder R^{e} 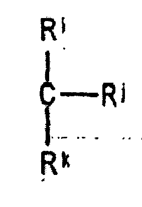 ist,
wobei Rⁱ, R^{j} und R^{k} unabhängig ausgewählt sind aus H, einer linearen oder verzweigten, gesättigten oder ungesättigten C₁-₆Alkyl-Kette, die wahlweise durch ein oder zwei Heteroatome ausgewählt aus N, O und S unterbrochen ist; und
die wahlweise substituiert ist durch einen oder mehrere Substituenten; Alkoxycarbonyl, Aminocarbonyl, Alkoxy, einen wahlweise substituierten 3- bis 6-gliedrigen heterocyclischen oder carbocyclischen Ring; mit der Maßgabe, dass zumindest eines von Rⁱ, R^{j} und R^{k} nicht H ist;
oder R^{e} 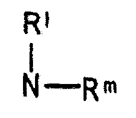 ist,
wobei R^{l} und R^{m} unabhängig ausgewählt sind aus H, einer linearen oder verzweigten, gesättigten oder ungesättigten C₁₋₆Alkyl-Kette, die wahlweise durch ein oder zwei Heteroatome ausgewählt aus N, O und S unterbrochen ist; und wahlweise substituiert ist durch einen oder mehrere Substituenten; Alkoxycarbonyl, Aminocarbonyl, Alkoxy, einen wahlweise substituierten 3- bis 6-gliedrigen heterocyclischen oder carbocyclischen Ring; mit der Maßgabe, dass zumindest eines von R^{l} und R^{m} nicht H ist;
oder R^{e} 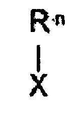 ist,
wobei X aus O oder S ausgewählt und Rⁿ aus einer linearen oder verzweigten, gesättigten oder ungesättigten C₁₋₆AlkylKette ausgewählt ist, die wahlweise durch ein oder zwei Heteroatome ausgewählt aus N, O und S unterbrochen ist; und wahlweise substituiert ist durch einen oder mehrere Substituenten; Alkoxycarbonyl, Aminocarbonyl, Alkoxy, und einen wahlweise substituierten 3- bis 6-gliedrigen heterocyclischen oder carbocyclischen Ring;
R^{h} aus Hydroxyl, Alkoxy, einem Aminosäurerest, einer Verbindungsgruppe und einem Targetingmolekül ausgewählt ist; und wobei das Metallzentrum aus Technetium oder Rhenium ausgewählt ist.

3. Eine Verbindung, welche nach Koordination an ein Metallzentrum ein einzelnes Stereoisomer ausgewählt aus:
Dimethylgly-L-*t*-Butylgly-L-Cys-Gly;
Dimethylgly-D-*t*-Butylgly-L-Cys-Gly;
Dimethylgly-L-*t*-Butylgly-L-Cys;
Dimethylgly-L-*t*-Butylgly-L-Cys(S^{ε}-Acm)-Gly-Thr-Lys-Pro-Pro-Arg;
bildet, wobei das Metallzentrum aus Technetium oder Rhenium ausgewählt ist.

4. Eine Verbindung nach einem der Ansprüche 1 bis 3 in einer mit einem Metall oder Metallradionuklid oder einem Oxid oder Nitrid davon komplexierten Form, wobei das Metall aus Technetium oder Rhenium ausgewählt ist.

5. Eine pharmazeutische Zusammensetzung, welche einen pharmazeutisch akzeptablen Träger und eine Verbindung, wie sie in Anspruch 4 definiert ist, in einer Menge umfasst, die effektiv ist, um eine Stelle von diagnostischem Interesse bildlich darzustellen.

6. Eine pharmazeutische Zusammensetzung wie in Anspruch 5 definiert, zur Verwendung bei der Strahlen-Bildgebung ("Radioimaging") einer Stelle von diagnostischem Interesse.

## Revendications

1. Composé qui forme principalement un seul stéréoisomère après coordination avec un centre métal de formule I suivants : 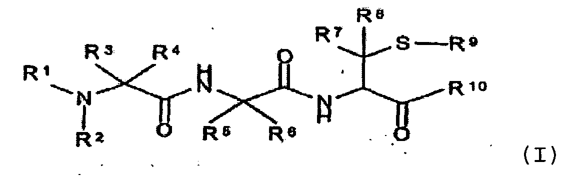 dans laquelle
R¹ est une chaîne alkyle en C₁-C₄, linéaire ou ramifiée, saturée ou insaturée qui est facultativement interrompue par ou deux hétéroatomes choisis parmi N, O et S; et est facultativement substituée par un ou plusieurs substituants choisis parmi halogène, hydroxyle, amino, carboxyle, alkyle en C₁-C₄, aryle et C(O)R¹⁰;
R² est H ou un substituant défini par R^{1;}
R¹ et R² peuvent former conjointement un hétérocycle de 5 à 8 chaînons, saturé ou insaturé, facultativement substitué par un ou plusieurs substituants choisis parmi halogène, hydroxyle, amino, carboxyle, oxo, alkyle en C₁-C₄ et aryle;
R³, R⁴ et R⁵ sont choisis indépendamment parmi H; carboxyle; alkyle en C₁-C₄; alkyle en C₁-C₄ substitué avec un substituant choisi parmi hydroxyle, amino, sulfhydryle, halogène, carboxyle, alkoxycarbonyle en C₁-C₄ et aminocarbonyle; une chaîne latérale de carbone alpha d'un acide D- ou L-aminé autre que la proline; et C(O)R¹⁰;
R⁶ est choisi parmi le groupe constitué de :
i) un hétérocycle ou un carbocycle de 3 à chaînons facultativement substitué;
ii) un composé ayant la formule suivants : 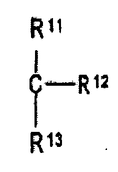 dans laquelle R¹¹, R¹² et R¹³ sont indépendamment choisis parmi H, une chaîne alkyle en C₁-C₆, linéaire ou ramifiée, saturée ou insaturée, qui est facultativement interrompue par un ou deux hétéroatomes choisis parmi N, 0 et S; et est facultativement substituée par un ou plusieurs substituants choisis parmi alkoxycarbonyle, aminocarbonyle, alkoxy, un hétérocycle ou un carbocycle de 3 à 6 chaînons facultativement substitué; à condition qu'au moins un parmi R¹¹, R¹² et R¹³ ne soit pas H;
iii) un composé de formule suivante : 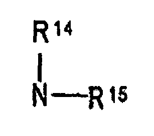 dans laquelle R¹⁴ et R¹⁵ sont indépendamment choisis parmi H, une chaîne alkyle en C₁-C₆, linéaire ou ramifiée, saturée ou insaturée, qui est facultativement interrompue par un ou deux hétéroatomes choisis parmi N, 0 et S; et est facultativement substituée par un ou plusieurs substituants; alkoxycarbonyle, aminocarbonyle, alkoxy, un hétérocycle ou carbocycle de 3 à 6 chaînons facultativement substitué; à condition qu'au moins un parmi R¹⁴ et R¹⁵ ne soit pas H; et
iv) un composé de formule suivants : 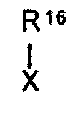
dans laquelle X est choisi parmi O ou S et R¹⁶ est choisi parmi une chaîne alkyle en C₁-C₆, linéaire ou ramifiée, saturée ou insaturée, qui est facultativement interrompue par un ou deux hétéroatomes choisis parmi N, 0 et S; et est facultativement substituée par un ou plusieurs substituants choisis parmi alkoxycarbonyle, aminocarbonyle, alkoxy, et un hétérocycle ou carbocycle de 3 à 6 chaînons facultativement substitué;
R⁷ et R⁸ sont choisis indépendamment parmi H; carboxyle; amino; alkyle en C₁-C₄; alkyle en C₁-C₄ substitué par un substituant choisi parmi hydroxyle, carboxyle et amino; et C(O)R¹⁰;
R⁹ est choisi parmi H et un groupement protecteur du soufre; et
R¹⁰ est choisi parmi hydroxyle, alkoxy, un résidu d'acide aminé, un groupement de liaison et une molécule ciblante; et
dans lequel le centre métal est choisi parmi le technétium ou le rhénium.

2. Composé qui forme principalement un seul stéréoisomère après coordination avec un centre métal de formule II suivante : 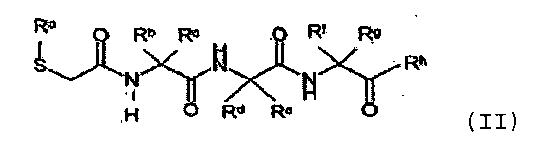 dans laquelle
R^{a} est choisi parmi H et un groupement protecteur du soufre;
R^{b}, R^{c}, R^{d}, R^{f} et R^{g} sont choisis indépendamment parmi H; carboxyle; alkyle en C₁-C₄; alkyle en C₁-C₄ substitué avec un substituant choisi parmi hydroxyle, amino, sulfhydryle, halogène, carboxyle, alkoxycarbonyle en C₁-C₄ et aminocarbonyle; une chaîne latérale de carbone alpha d'un acide D- ou L-aminé autre que la proline; et C(O)R^{h};
R^{e} est un hétérocycle ou carbocycle de 3 à 6 chaînons facultativement substitué;
ou R^{e} est 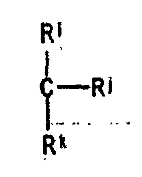 dans laquelle Rⁱ, Rⁱ et R^{k} sont indépendamment choisis parmi H, une chaîne alkyle en C₁-C₆, linéaire ou ramifiée, saturée ou insaturée, qui est facultativement interrompue par un ou deux hétéroatomes choisis parmi N, 0 et S; et est facultativement substituée par un ou plusieurs substituants; alkoxycarbonyle, aminocarbonyle, alkoxy, un hétérocycle ou carbocycle de 3 à 6 chaînons facultativement substitué; à condition qu'au moins un parmi R¹, R^{j} et R^{k} ne soit pas H;
ou R^{e} est 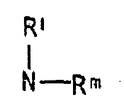 dans laquelle R^{l} et R^{m} sont indépendamment choisis parmi H, une chaîne alkyle en C₁-C₆, linéaire ou ramifiée, saturée ou insaturée, qui est facultativement interrompue par un ou deux hétéroatomes choisis parmi N, 0 et S; et est facultativement substituée par un ou plusieurs substituants; alkoxycarbonyle, aminocarbonyle, alkoxy, un hétérocycle ou carbocycle de 3 à 6 chaînons facultativement substitué; à la condition qu'au moins un parmi R^{l} et R^{m} ne soit pas H;
ou R^{e} est 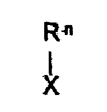 dans laquelle X est choisi parmi 0 ou S et Rⁿ est choisi parmi une chaîne alkyle en C₁-C₆, linéaire ou ramifiée, saturée ou insaturée, qui est facultativement interrompue par un ou deux hétéroatomes choisis parmi N, 0 et S; et est facultativement substituée par un ou plusieurs substituants; alkoxycarbonyle, aminocarbonyle, alkoxy, et un hétérocycle ou carbocycle de 3 à 6 chaînons facultativement substitué;
R^{h} est choisi parmi hydroxyle, alkoxy, un résidu d'acide aminé, un groupement de liaison et une molécule ciblante; et
dans lequel le centre métal est choisi parmi le technétium ou le rhénium.

3. Composé qui forme principalement un seul stéréoisomère après coordination avec un centre métal choisi parmi :
Diméthylgly-L-t-Butylgly-L-Cys-Gly;
Diméthylgly-D-t-Butylgly-L-Cys-Gly;
Diméthylgly-L-t-Butylgly-L-Cys;
Diméthylgly-L-t-Butylgly-L-Cys(S^{ε}-Acm)-Gly-Thr-Lys-Prn-Pro-Arg;
où le centre métal est choisi parmi le technétium ou le rhénium.

4. Composé selon l'une quelconque des revendications 1 à 3 dans une forme complexée avec un métal ou un radionucléide métallique ou un oxyde ou nitrure de ceux-ci, dans lequel le métal est choisi parmi le technétium ou le rhénium.

5. Composition pharmaceutique comprenant un support pharmaceutiquement acceptable et un composé selon la revendication 4 dans une quantité efficace pour visualiser un site d'intérêt diagnostique.

6. Composition pharmaceutique selon la revendication 5 pour une utilisation dans la radio-imagerie d'un site d'intérêt diagnostique.
